# EUROPÄISCHE PATENTANMELDUNG

(11) **EP 0 970 673 A2**
(43) Veröffentlichungstag der Anmeldung: **12.01.2000**
(21) Anmeldenummer: 99108457.5
(22) Anmeldetag: 30.04.1999
(51) Int. Cl.: A61F 13/00

(54) **Pflaster zur Abdeckung insbesondere des Randbereiches künstlicher Körperöffnungen**

(30) Priorität: 08.07.1998 DE 19830421
(71) Anmelder: Resorba Chirurgisches Nahtmaterial Franz Hiltner GmbH & Co., 90443 Nürnberg (DE)
(72) Erfinder: Hiltner, Claus Martin, D-90408 Nürnberg (DE)
(74) Vertreter: Schneck, Herbert, Dipl.-Phys., Dr.

(57) **Zusammenfassung**

Bei einer Einrichtung zur Abdeckung insbesondere des Randbereiches künstlicher Körperöffnungen, durch welche externe Fixateure, Halteschrauben, Katheter, Drainageschläuche, elektrische Leitungen oder dergleichen von außen in das Körperinnere geführt werden, ist vorgesehen, daß die Abdeckung im wesentlichen scheibenförmig ist, eine Durchtrittsöffnung aufweist und zur Aufnahme eines desinfizierenden Agens, z.B. in Form eines Gels, Hydrogels, Kolloidgels oder eine Paste, ausgebildet ist.

## Beschreibung

Die Erfindung richtet sich aufeine Einrichtung zur Abdeckung insbesondere des Randbereiches künstlicher Körperöffnungen, durch welche externe Fixateure, Halteschrauben. Katheter, Drainageschläuche, elektrische Leitungen oder dergleichen von außen in das Körperinnere geführt werden.

Bei der Versorgung von Knochenbrüchen werden beispielsweise sogenannte externe Fixateure, d.h. Halteschienen aus Edelstahl, eingesetzt. Mit diesen Geräten wird die zur Schienung der Knochenbruchstelle notwendige Fixierung außerhalb des Weichteilgewebes verlegt. Die Verbindung zu den Knochenfragmenten geschieht über Verbindungsschrauben zwischen Fixateur und Knochen. Es ist von Vorteil, die Fixierschiene nicht knochennah im Gewebe zu verlegen und damit traumatische Eingriffe beim Setzen bzw. Entfernen der Metallteile zu verursachen. Dieser Vorteil geht mit einem niedrigeren Infektionsrisiko während der Operation, jedoch auch mit einer höheren Gefahr eines posttraumatischen Eindringens von Keimen während der Heilungsphase über den Randbereich zwischen künstlicher Körperöffnung und Fixateur-Schraube einher.

Die Eintrittsstelle des Fixateur-Pins kann während der gesamten Knochenheilungsphase als offener Übergangsbereich von der Haut zum Fixateurstahl nicht verheilen und muß aufgrund des besonderen Infektionsrisikos gewissenhaft gepflegt, d.h. gereinigt und desinfiziert werden. Im allgemeinen wird dazu regelmäßig während der gesamten Tragezeit eine jodhaltige Lösung auf die Eintrittsstelle aufgebracht.

Aufgrund mangelnder Sorgfalt bei der Pflege bzw. ungünstiger Lage der Eintrittsstellen kommt es dennoch immer wieder zu Infektionen. In das Weichgewebe eindringende Keime können in seltenen Fällen bis zum Knochen vordringen und sogar diesen infizieren. Solche Infektionen, wie z.B. Osteomyeliten, sind für den Patienten häufig mit einer langwierigen, schwierigen Therapie mit erheblicher körperlicher Beeinträchtigung verbunden.

Neben der Gefahr einer Kontamination der Wunde während der Ausheilungsphase kommt es am Übergang von Weichteilgewebe zur Stahloberfläche zu einer permanenten Reizung und damit zur Entzündung der Eintrittsstelle. Eine negative Beeinflussung des kosmetischen Ergebnisses der späteren Hautwundheilung durch verstärkte Narbenbildung kann die Folge sein.

Ein anderer Problemfall der in Betracht stehenden Art ist die Anwendung einer Katheterbehandlung, z.B. bei der Peritoneal-Dialyse, wobei über eine längere Zeitspanne ein Kathetereintritt in den Bauchraum gegeben ist. Es liegt ein nicht physiologischer Übergang von Weichteilgewebe zu einem Kunststoff-Schlauch an der Körperoberfläche vor.

Dieser Bereich stellt auch hier einen durch Keimkontamination gefährdeten Hautbereich dar, und zwar insbesondere durch die Überleitung aufder Kunststoffoberfläche des Katheters als Verbindung zum Peritoneum. Eine Infektion im Körperinneren, sogenannte Peritonitis, infolge des Passierens von Luftkeimen oder Oberflächenkeimen über diese Eintrittsstelle ist deshalb nicht selten.

Weiterhin ist für die erfindungsgemäße Anwendung auch der Fall zu betrachten, wo Katheterschläuche zu Drainagezwecken in das Körperinnere eingeführt werden, um Flüssigkeitsansammlungen, z.B. im Thoraxraum, zu entfernen. Solche Drainagen werden über mehrere Tage hin aufrechterhalten und sind ungeachtet der therapeutischen Erfolge ein Pflegeproblem aufgrund der Mobilität des Patienten.

Hiervon ausgehend liegt der Erfindung die Aufgabe zugrunde, eine Einrichtung zu schaffen, die eine gezielte Behandlung und Pflege des Randbereichs von Körperöffnungen der vorstehend erläuterten Art ermöglicht.

Diese Aufgabe wird gelöst durch eine Einrichtung zur Abdeckung des Randbereiches solcher künstlicher Körperöffnungen, durch welche externe Fixateure, Halteschrauben, Katheter, Drainageschläuche, elektrische Leitungen oder dergleichen von außen in das Körperinnere geführt werden, wobei die Abdeckung im wesentlichen scheibenförmig ist, eine Durchtrittsöffnung aufweist und zur Aufnahme eines desinfizierenden Agens, z.B. in Form eines Gels, Hydrogels, Kolloidgels oder einer Paste ausgebildet ist.

Hierdurch wird der für die Pflege kritische Bereich zwischen Patientenhaut und Gegenstand als mögliche Eintrittsstelle für Keime besonders geschützt. Die Eintrittsstelle des Gegenstands in das Hautgewebe wird feucht gehalten, während sich die Wirkung des aus dem Gel herausdifundierenden Antiseptikums verstärkt. Wahlweise kann auch aufden Einsatz einer jod- oder silberhaltigen Lösung bzw. eines Antibiotikums zurückgegriffen werden.

Vorzugsweise ist die Abdeckung als Kunststoffscheibe bzw. Kunststoffkörper ausgebildet, die günstigerweise einen schaumstoffbeschichteten Rand aufweist, so daß ein Innenraum oder Hohlraum für die Aufnahme des Agens ausgebildet wird.

Die Scheibe ist mit Vorteil mit einer Klebeschicht versehen, so daß eine Befestigung nach Art eines Pflasters auf der Hautoberfläche möglich ist.

Ein sich günstigerweise von der Durchgangsöffnung zum Außenrand erstreckender Öffnungsschlitz ermöglicht es, die Abdeckung bei der ersten Anbringung oder beim Austausch seitlich aufzuschieben, ohne daß der die Haut durchsetzende Gegenstand entfernt werden muß.

Nachfolgend wird die Erfindung anhand eines bevorzugten Ausführungsbeispieles in Verbindung mit der Zeichnung näher erläutert. Dabei zeigen:

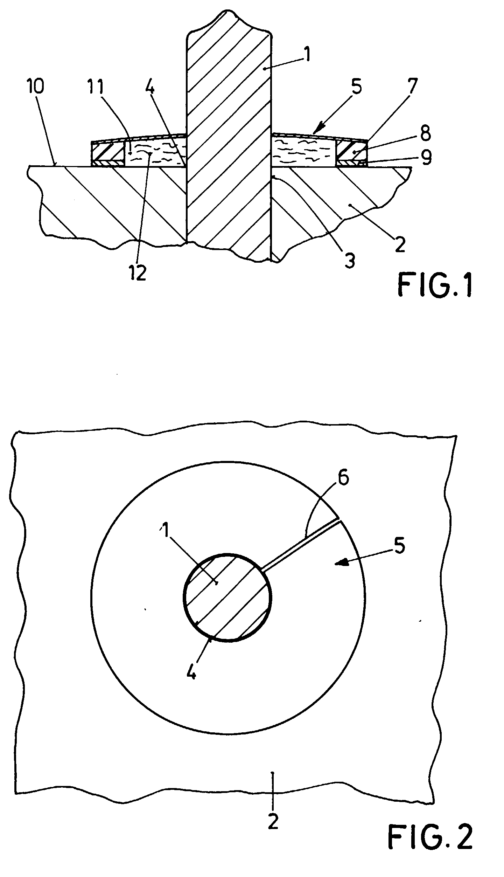

- Fig. 1: einen Schnitt durch den Eintrittsbereich eines externen Fixateurs mit einer erfindungsgemäßen Abdeckeinrichtung und
- Fig. 2: eine Aufsicht auf diesen Bereich.

Ein Pin 1 eines sogenannten externen Fixateurs ist zur Fixierung eines in der Zeichnung nicht dargestellten Knochens in eine menschliche Extremität 2, beispielsweise ein Bein, eingesetzt. Hierzu weist die Extremität 2 eine Öffnung 3 mit einem Randbereich 4 auf.

Dieser Randbereich ist eine, wie vorstehend bereits geschildert, kritische Zone.

Eine erfindungsgemäße Abdeckeinrichtung 5 aus einem Kunststoff-Plättchen ist mit einem Öffnungsschlitz 6 versehen und kann dementsprechend seitlich unter Aussparung des Pins 1 über diesen geschoben werden, so daß der Randbereich 4 von der Abdeckeinrichtung 5 abgedeckt wird.

Der Öffnungsschlitz 6 kann bei Bedarf mit einer selbstklebenden Abdecklasche zum Schutz des Wirkstoffgels gegen Austrocknung versehen werden.

Die Abdeckeinrichtung 5 weist längs ihres Außenrandes 7 einen Schaumstoff-Streifen 8 auf, der an seiner Unterseite mit einer doppelseitigen Klebefolie 9 versehen ist so daß ein Festkleben aufder Hautoberfläche 10 der Extremität 2 möglich ist.

Im Inneren des Schaumstoff-Streifens 8 wird dementsprechend ein Raum 11 ausgebildet, der zur Aufnahme eines insbesondere antiseptischen Agens 12 geeignet ist.

Anstelle des Schaumstoff-Streifens 8 kann vorgesehen sein, daß die Abdeckeinrichtung als tiefgezogenes Kunststoffteil ausgebildet ist und die Funktion des Schaumstoff-Streifens durch einen topfartigen Wandabsehnitt der Abdeckung realisiert wird.

Über die in der Zeichnung dargestellte Ausführungsform hinaus kann vorgesehen sein, daß die Abdeckeinrichtung 5 einen Ringverschluß aufweist, dessen Innenseite rauh ausgebildet bzw. mit Zähnen besetzt ist, so daß hierdurch auch eine Haltefunktion für Katheterschläuche oder dergleichen erreichbar ist.

## Patentansprüche

1. Einrichtung zur Abdeckung insbesondere des Randbereiches künstlicher Körperöffnungen, durch welche externe Fixateure, Halteschrauben, Katheter, Drainageschläuche, elektrische Leitungen oder dergleichen von außen in das Körperinnere geführt werden, **wobei** die Abdeckung im wesentlichen scheibenförmig ist, eine Durchtrittsöffnung aufweist und zur Aufnahme eines desinfizierenden Agens, z.B. in Form eines Gels, Hydrogels, Kolloidgels oder eine Paste, ausgebildet ist.

2. Einrichtung nach Anspruch 1, **dadurch gekennzeichnet, daß** sie ein Kunststoffkörper ist.

3. Einrichtung nach Anspruch 1, **dadurch gekennzeichnet, daß** sie einen schaumstoffbeschichteten Rand aufweist.

4. Einrichtung nach Anspruch 3, **dadurch gekennzeichnet, daß** sie mit einer Klebeschicht versehen ist.

5. Einrichtung nach Anspruch 1, **dadurch gekennzeichnet, daß** sich von der Durchgangsöffnung zum Außenrand ein Öffnungsschlitz erstreckt.
